# EUROPEAN PATENT APPLICATION

(11) **EP 3 225 151 A1**
(43) Date of publication of application: **04.10.2017**
(21) Application number: 17164275.4
(22) Date of filing: 31.03.2017
(51) Int. Cl.: A61B 1/00, A61B 1/05, A61B 1/06, A61B 5/00, A61B 5/107

(54) **THORACIC ENDOSCOPE FOR SURFACE SCANNING**

(30) Priority: 31.03.2016 US 201662315773 P; 24.03.2017 US 201715468981
(71) Applicant: Covidien LP, Mansfield, MA 02048 (US)
(72) Inventor: SARTOR, Joe D., Longmont, CO Colorado 80504 (US); HARDING, William C., Chandler, AZ Arizona 85248 (US); DIGMANN, Patrick, Louisville, CO Colorado 80027 (US)
(74) Representative: Maschio & Soames IP Ltd

(57) **Abstract**

A surgical apparatus is provided including an endoscope, a camera, a light source, and a structured light pattern source. The endoscope includes an elongate body having a plurality of segments manipulatable relative to one another. The camera, light source, and structured light pattern source cooperate to determine the topography of a surface within a patient. A method of performing surgery is also provided.

## Description

### BACKGROUND

### Technical Field

The present disclosure relates to surgical systems, and more particularly, to systems and methods of performing endoscopic thoracic imaging and treatment.

### Description of Related Art

It is a daily occurrence for people to enter a medical facility in order to be diagnosed or treated by clinician for a multitude of different medical conditions. Paramount to proper treatment and diagnosis in some instance is the clinician's ability to target and sufficiently access the area of interest. Additionally, in most circumstances, clinician's strive to minimize the invasiveness of the medical procedure. This goal of minimal invasiveness limits a clinician's options in accessing the area of interest, and thus medical procedures and medical tools have been developed accordingly. Common methods for a minimally invasive treatment are surgeries performed using one or more access ports enabling the insertion of tools (e.g., graspers and ligation tools) as well as optics enabling the clinician to view the area being treated. Access ports come in a variety of styles and mechanisms using bladed, bladeless, and blunt obturator type trocars. Access ports often have at least one cannula enabling the insertion of tools and optics there through. The trocars are inserted into the cannula and the combination is inserted through a small opening or incision on the patient. Once placed, the trocar is removed from the cannula leaving the cannula available for the insertion of tools. For a given application the trocar and cannulas may be formed of stainless steel, plastics, and a combination of the two.

One specialty access port that is often used for "single port" surgeries are marketed by Medtronic under the name SILS™ Ports. SILS™ Ports are surgically inserted in the umbilicus of the patient and are formed of an elastomeric material. In one example, three cannulas transcend the port, enabling the insertion of three different tools through a single opening in the patient, which is in a location where it will leave little or no observable scarring. Indeed, 'single port' approaches to laparoscopy are major advances because of the limited number of incisions and thus decreased 'invasiveness' quotient which generally improves outcomes for the patient.

A well-known laparoscopic thoracic surgery is the video-assisted thoracoscopic surgery (VATS). Typically during a VATS, a patient is intubated with a double lumen endotracheal tube, with each lumen orientated towards a different lung. In this manner, a clinician may induce atelectasis in the lung to be treated and provide proper ventilation to the untreated lung. Following the placement of the double lumen endotracheal tube, the clinician creates one or more incisions in the chest wall. Alternatively, a clinician may insert access ports should insufflation be needed to reduce the lung volume; as is more commonly required with patients having chronic obstructive pulmonary disease (COPD). Commonly, a clinician will make one large incision at the 4 or 5^{th} intercostal space and typically up to three more incisions, as needed, to achieve access and triangulation between cameras and instruments. In some instances the chest wall may be pierced by an insufflation needle prior to the incisions. The typical size of an incision ranges about from 2 centimeters to about 6 centimeters. The exact placement of the incisions depends upon the area of the lung that the clinician is seeking to access, but generally each incision will be placed within the intercostal space (i.e., a space between two of the patient's ribs and in a complementary position to one another). The clinician can then place the access ports in each incision relying on the trocar to enlarge or create an opening into which the cannula will rest at the completion of the insertion.

The clinician will generally select one of the access ports for the insertion of a surgical camera and will select the other access port for the insertion of surgical devices. In some instances, the camera may be inserted into the trocar prior to insertion to enable the clinician to observe the insertion process. The use of each access port may be interchangeable throughout the procedure. The camera inserted through the selected port transmits images of inside the patient's thoracic cavity onto a video monitor, providing guidance for the clinician. Once the clinician has located the area of interest using the surgical camera, surgical devices are inserted and navigated through respective access ports to undertake the necessary treatments. After the treatment is completed, the camera and surgical devices are removed, the access ports are removed, and the incisions in the patient closed.

Another minimally invasive approach is the use of endoscopy to reach a desired location within the body. Though not exclusively, endoscopic approach are often employed in diagnostic (e.g., biopsy) procedures, to eliminate the need for making an insertion into a patient, though endoscopes can and are inserted into a patient via a small incision in certain instances. While endoscopes are often utilized to access luminal tissue via a natural orifice (e.g., nose, mouth or anus), they can also be inserted into an access port.

Though current endoscopes are useful for their current tasks and quite useful in navigating to tissue for inspection and treatment, improvements are always desirable and sought after.

### SUMMARY

The present disclosure is directed to a surgical apparatus including an endoscope, a camera, a light source, and a structured light pattern source. The endoscope includes an elongate body having a plurality of segments that are manipulatable relative to one another. The camera, the light source, and the structured light pattern source cooperate to determine the topography of a surface within a patient.

In aspects, the structured light pattern source may be a structured light scanning laser. In certain aspects, the structured light scanning laser may be an LED laser having collimated light.

In other aspects, the surgical apparatus may include a second camera that is configured to intercept reflected light from the structured light scanning laser, the collimated light being in the infrared or visible spectrum.

In certain aspects, a distal-most segment of the endoscope may terminate at a distal face, wherein the camera, light source, and second camera are disposed within the distal face and the structured light scanning laser is disposed on an outer surface of the distal-most segment.

In aspects, the structured light scanning laser and the second camera may be oriented at an angle relative to a centerline defined by the distal-most segment.

In other aspects, the structured light pattern source may be a digital light processing system. In certain aspects, the digital light processing system may include a light source, at least one mirror, a first lens, a digital light processing chip, and a second lens.

In other aspects, the digital light processing system and the camera may be disposed within the distal-most segment of the endoscope. In aspects, the digital light processing system may be disposed external to the distal-most segment of the endoscope and the camera may be disposed within the distal-most segment of the endoscope.

In certain aspects, the digital light processing system and the camera may be disposed external to the distal-most segment of the endoscope. In other aspects, the surgical apparatus may include a second endoscope, wherein the digital light processing system is coupled to the endoscope and the camera is coupled to the second endoscope.

According to another aspect of the present disclosure, a method of performing surgery is provided. The method includes storing instructions on a memory associated with a computer, advancing an endoscope within a body cavity of a patient, manipulating a distal segment of a plurality of segments defined by the endoscope towards a target tissue, projecting a structured light pattern from a structured light pattern source that is coupled to the distal segment, detecting portions of the structured light pattern that is reflected off of the target tissue using a camera coupled to the distal segment, and executing the instructions to generate a three dimensional map of the target tissue using data obtained by the camera.

In aspects, projecting a structured light pattern from a structured light pattern source may include projecting a structured light pattern from a structured light scanning laser. In certain aspects, detecting portion of the structured light pattern may include detecting portions of the structured light pattern using a second camera coupled to the distal segment.

In other aspects, projecting a structured light pattern from a structured light pattern source may include projecting a structured light pattern from a digital light processing system. In aspects, the method may include correlating images obtained by an optical camera coupled to the distal segment with images of a patient stored on the memory.

In certain aspects, the method may include tracking a location of the endoscope within the body cavity of a patient. In other aspects, executing the instructions to generate a three dimensional map of the target tissue may include utilizing the correlated images and the tracked location of the endoscope to generate an accurate three dimensional map of the target tissue.

### BRIEF DESCRIPTION OF THE DRAWINGS

Various aspects and features of the present disclosure are described hereinbelow with references to the drawings, wherein:
FIG. 1 is a side, cross-sectional, view of the thoracic cavity of a patient with an endoscope having surface scanning capabilities provided in accordance with the present disclosure advanced therein;
FIG. 2 is a front, perspective view, of the endoscope of FIG. 1;
FIG. 2A is a front, perspective view, of an alternative embodiment of the endoscope of FIG. 1;
FIG. 3 is a front, perspective view, of another embodiment of an endoscope having surface scanning capabilities provided in accordance with the present disclosure;
FIG. 4 is a front, perspective view, of yet another embodiment of an endoscope having surface scanning capabilities provided in accordance with the present disclosure;
FIG. 5 is a side, cross-sectional, view of the thoracic cavity of a patient with another endoscope having surface scanning capabilities provided in accordance with the present disclosure advanced therein;
FIG. 6 is side, cross-sectional, view of a distal end portion of the endoscope of FIG. 5;
FIG. 7 is a side, cross-sectional, view of a distal end portion of the endoscope of FIG. 5 shown with a structured light image being projected therefrom;
FIG. 8 is a side view of another embodiment of an endoscope having surface scanning capabilities;
FIG. 9 is a side view of yet another embodiment of an endoscope having surface scanning capabilities; and
FIG. 10 is a schematic illustration of a robotic surgical system configured for use in accordance with the present disclosure.

### DETAILED DESCRIPTION

One aspect of the present disclosure is directed to an endoscope and systems that support organ matching to preoperative images, for example images of lung. The endoscope can provide both visual imaging and also surface mapping. Typically, optical contrast of the lung is very low due to the mottled color ranging from pink to gray. This makes optical surface mapping using 3D cameras very challenging. In accordance with the present disclosure the endoscope includes a structured light (or laser) scanner. As can be appreciated, the structured light scanner may employ infrared light so as to avoid interference from visible light sources, although it is contemplated that the structured light scanner may emit light in the visible spectrum, depending upon the tissue being scanned during the procedure. The structured light source includes a known position relative to a camera and permits the calculation of the exact location of the intersection between the light ray from the structured light source and the camera. This information can be scanned as single points, lines, or arrays to create topologic maps of surfaces. In embodiments, the structured light source is that of an LED or LED infrared laser that is dispersed into a scan pattern (line, mesh, or dots), buy rotating mirror, beam splitter, or diffraction grating. In one non-limiting embodiment, the structured light source may be a LED Laser having collimated light. The laser scanner will enable visualization systems to achieve accurate surface maps of the lung needed in order to match preoperative computed images to the operative image delivered to the endoscopic camera. Having both in one endoscope offers additional advantage of matching the preoperative computed image to the current camera view as the camera offset is known relative to the surface scan.

Additionally, the structured light source may be a digital light processing (DLP) projection system having a light source, a first lens, one or more mirrors, a DLP chip, and a second lens. A camera is spaced apart and angled relative to the second lens at known values such that the topography of the surface of the lungs may be determined using trigonometric functions. In embodiments, the DLP projection system is located external to the patient. The structured light pattern is transmitted through the endoscope via a light guide cable and the reflected image is captured by a camera disposed on a distal end portion of the endoscope. Alternatively, both the DLP projection system and the camera may be disposed external to the patient. In this manner, a first endoscope is utilized to transmit the structured light pattern and a second, separate and distinct, endoscope is utilized to transmit the images captured by the camera.

In particular applications the endoscope position will also be tracked by intraoperative instrument tracking systems for example electromagnetic navigation such as those described in PCT International Application No. PCT/US16/30028 filed on April 29, 2016, SYSTEMS AND METHODS FOR CONTROLLING AN ELECTROSURGICAL GENERATOR DURING A MICROWAVE ABLATION PROCEDURE to Girotto, the entire contents of which is incorporated herein by reference. The locational information obtained by the intraoperative instrument tracking system aids in simplifying the algorithms needed to produce large scale surface maps from segmental sized scans taken from an endoscope. Further, this immediate intraoperative guidance of the optical image location to the surface map and preoperative computed images provides even greater clarity of location and orientation of the endoscope.

In certain embodiments, the endoscope is positionable by a robotic system. The robotic system provides precise six axis orientation of the endoscope in a similar manner to the navigation systems, but benefited by active positioning as well as locational knowledge of the endoscope within the patient. As can be appreciated, the robot may be utilized to autonomously move the endoscope to complete scans of larger areas or whole organs.

In one embodiment the endoscope will have four specific capabilities; a visual- light optical camera, a light source of preferably at least one light-emitting diode (LED), a scanning laser, and a second camera used to map the laser. In some embodiments, the scanning laser may be detected by the same optical camera in near to mid infrared imaging as optical sensor technology continues to advance. In its simplest form, as detailed below, the endoscope uses a typical arrangement of these components on the distal end of the endoscope. In order to reduce the required distal end diameter of the instrument and to improve triangulation between the laser and the second camera, these four components may have a location on at least one extensible surface. This enables the four components to be arranged along the side of the extensible surface such that the needed space for the individual components is provided by having a cross section equal or slightly larger than any single component and sufficient length to align the components side by side.

The computation of the topology viewed by the endoscope may require a calibration source to detail the alignment of the laser with the second camera. Anticipated is that the calibration may be conducted at the time of manufacture and stored within a memory coupled to a suitable computer, as will be described in detail hereinblow, or by targeting a calibration surface at the time of use. The calibration will be used internally with the device anticipating the computational topology that may be created with the endoscope and transmitted for clinician via common video transmission means or the raw camera data along with the calibration may be transmitted to an external graphics processor creating the computational topology.

In some embodiments, at least the laser and the second camera may be spaced along the length of the instrument shaft to enable triangulation where the laser and second camera are directed at an angle from the centerline of the instrument.

One can appreciate that if the components were on the outside diameter of the endoscope they might become immediately soiled and optically obstructed by passing through trocars. As such, in at least one embodiment the surface is protected by and an element opposing the surface housing the components while they pass through the trocar into the surgical field. This also enables the components to be constructed in a preferable configuration where components are opposing on two folding surfaces; the components being offset along the opposing surface (facing each other in the folded trocar insertion position) providing a minimum diameter of the endoscope not far exceeding the thickest (deepest component).

A further embodiment of the present disclosure is directed to an endoscope having folding faces to facilitate flow of liquid from the proximal to distal direction to flush and clean soil or blood from the components without withdrawing them from the observed positions.

Still a further aspect of the present disclosure is directed to its specific methods of thoracoscopic use of the endoscope. In one example, the space between the lung and the chest wall where the scope is used is often limited by shallow space in the anterior to posterior but lengthy in superior to inferior direction. As such, the scope may be designed to enter the chest and through multiple segments be able to hug the chest wall from the point of entry. This is shown as having a first bend or articulation at the distal end and a second bend 4cm proximal of the first bend. Progressively the scope may have additional bend point spaced proximally up the scope shaft.

Alternative to the first bend may have a hinge for the folding face not perpendicular to the endoscope axis such that as the faces fold outward they project the optical elements at an angle to the axes. This is somewhat equivalent to fixed angle optical endoscopes most often used at 30°.

The primary advantage of the present disclosure is to enable 3d surfacing of organs which can be matched to preoperative computational imaging needed for operative guidance to target lesions with particular special knowledge of adjacent structures and anatomic boundaries such as in sublobar resection or lung cancer. The particular folding configuration of the scope permits the forward facing surface containing the optical elements to be passed through a minimum diameter trocar or incision. Primary use for this system is thoracic but one can vision equal value in deep pelvic or rectal surgery. These and further aspects of the present disclosure are detailed herein below.

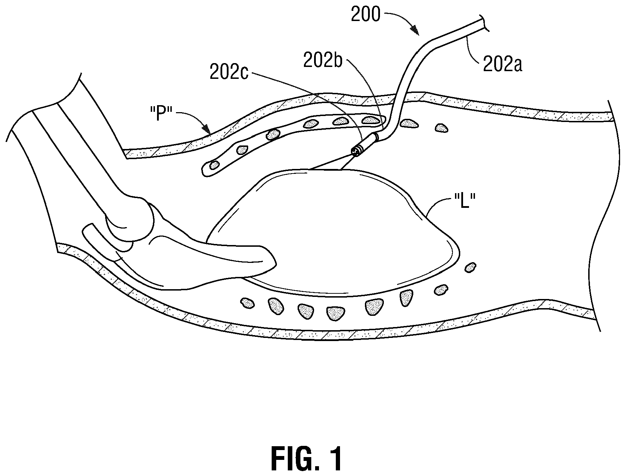

With reference to FIGS. 1-5, an endoscope having surface scanning capabilities provided in accordance with the present disclosure is illustrated and generally identified by reference numeral 200. The endoscope 200 includes an elongate body 202 configured to be advanced within a suitable thoracic trocar (not shown) or other device capable of penetrating the chest cavity and receiving an endoscope therein or a thoracic catheter or the like. In embodiments, the elongate body 202 may include first, second, and third segments 202a. 202b, 202c, respectively, each coupled to each other and capable of being manipulated relative to one another (FIG. 1). In this manner, the endoscope 200 may be positioned in close proximity to the chest wall to navigate the shallow portions of the thoracic cavity between the lungs "L" (FIG. 1) and chest wall of the patient "P". As can be appreciated, the elongate body may include any number of segments, such as four, five, six, etc. to aid in the maneuverability of the endoscope 200 within the thoracic cavity. Further, it is contemplated that the position at which the elongate body 202 may be bent to form each segment may be variable (i.e., the elongate body 202 may bend at any position along its length). In embodiments, each segment 202a, 202b, 202c may be individually manipulatable by a clinician by means of a handle or other suitable device in mechanical communication with each segment 202a, 202b, 202c.

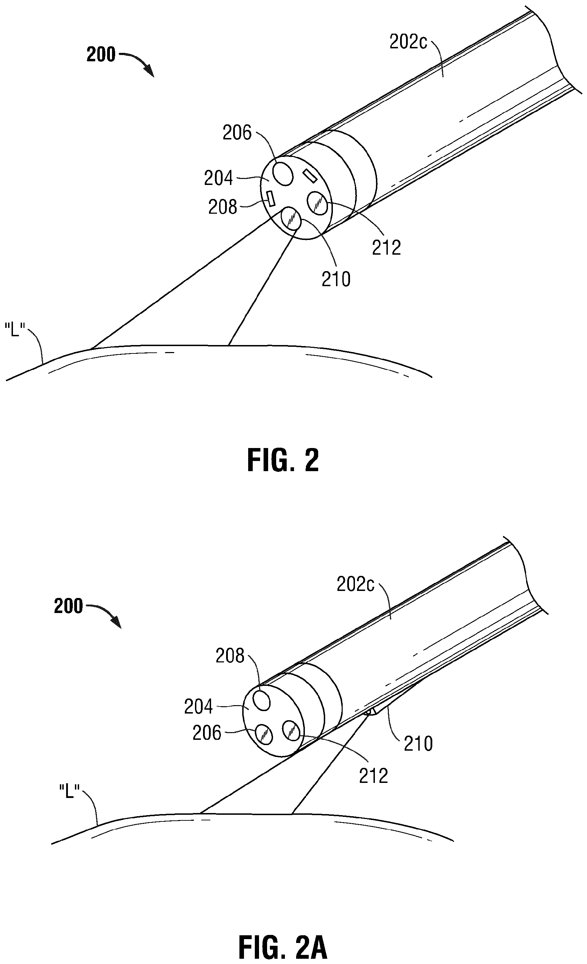

Referring to FIG. 2, the distal most segment, i.e., the third segment 202c, includes a distal surface 204. The distal surface 204 includes an optical camera 206, a light source 208, a structured light projection source or structured light scanner (laser) 210, and a second camera 212 disposed thereon. Although generally illustrated as being disposed in a circular configuration (i.e., disposed about the circumference of the distal surface 204), it is contemplated that each of the optical camera 206, light source 208, laser 210, and second camera 212 may be disposed in any suitable configuration. The optical camera 206 is a visual-light optical camera, such as a charge-coupled device (CCD), complementary metal-oxide-semiconductor (CMOS), N-type metal-oxide-semiconductor (NMOS), or other suitable camera known in the art. In one non-limiting embodiment, the optical camera 206 is a CCD camera having a resolution of 1080p. The light source 208 is a light emitting diode (LED) emitting white light, although any light emitting device known in the art may be utilized. The laser 210 is may be any structured light scanner known in the art, such as an LED or LED infrared laser that is dispersed into a scan pattern (line, mesh, or dots), by rotating mirror, beam splitter, or diffraction grating. In one non-limiting embodiment, the laser 210 is an LED laser having collimated light. The second camera 212 is a CCD camera capable of detecting IR light, although it is contemplated that the second camera 212 may detect visible light, such as visible green light or the like, depending upon the tissue being scanned. Specifically, visible green light contrasts with tissue having a red or pinkish hue enabling the second camera 212 to more easily identify the topography of the tissue. A digital filter (not shown) or a filter having narrow band optical grating (not shown) inhibits extraneous visible light emitted from the laser 210 from distracting the surgeon during the surgical procedure. In embodiments, the visible light is filtered from the image captured by the optical camera 206 and transmitted to the surgeon such that the image is clear and free from extraneous light patterns.

It is contemplated that the second camera 212 may be any thermographic camera known in the art, such as such as ferroelectric, silicon microbolometer, or uncooled focal plane array (UFPA), or may be any other suitable visible light camera such as a charge-coupled device (CCD), complementary metal-oxide-semiconductor (CMOS), N-type metal-oxide-semiconductor (NMOS), or other suitable camera known in the art where the light emitted from the laser 210 is in the visible spectrum. In embodiments, the distal surface 204 may include a suitable transparent protective cover (not shown) capable of inhibiting fluids or other contaminants from coming into contact with each of the optical camera 206, light source 208, laser 210, and second camera 212. Since the distance between the laser 210 and second camera 212 relative to the optical camera 206 is fixed (i.e., the offset of the optical camera 206 relative to the laser 210 and second camera 212), the images obtained by the optical camera 206 can more accurately be matched with a pre-operative image, as will be described in further detail hereinbelow. It is contemplated that the various sensors disposed within the distal end of the third segment 202c may be separate and distinct components with associated hardware and/or software, or may be part of a commercial platform such as Intel^{®}'s RealSense™.

In embodiments, the laser 210 may be disposed on an outer surface of the third segment 202c (FIG. 2A). As can be appreciated, the location of the laser 210 on the outer surface of the third segment 202c enables triangulation where the laser 210 and second camera 212 are directed at an angle from the centerline of the third segment 202c (i.e., the laser 210 and second camera 212 are disposed at an angle incident to a longitudinal axis defined by the third segment 202c). As the aperture of the laser diode is relatively small it may benefit by a cover not shown having hydrophobic properties by means of the material or well-known coatings such as silicones or HDMSO Plasma depositions. Also a slightly raised geometry shed body fluids or washes ejected onto the cover from an internal fluid path connected proximally to a fluid path not shown.

In operation, initially, the patient "P" (FIG. 1) is imaged using any suitable imaging device (not shown), such as MRI, ultrasound, CT scan, Positron Emission Tomography (PET), or the like, and the images are stored within a memory (not shown) coupled to a suitable computer (not shown). The memory may include any non-transitory computer-readably storage media for storing data and/or software that is executable by a processor (not shown) e.g., solid-state, volatile, non-volatile, removable, and non-removable.

After the patient "P" is imaged, the clinician penetrates the chest of a patient "P" using a trocar (not shown) or other suitable device. The third segment 202c of the endoscope 200 is advanced within the trocar, and thereafter, within the thoracic cavity of the patient "P" (FIG. 1). As the endoscope 200 is further advanced within the thoracic cavity, the clinician observes the images obtained by the optical camera on a display (not shown) and once the distal end of the third segment 202c of the endoscope is adjacent the lungs "L", the clinician manipulates the second segment 202b relative to the first segment 202a in order to orient the second and third segments 202b, 202c in a parallel orientation relative to the surface of the lungs "L" (FIG. 1). Once in a parallel orientation, the clinician manipulates the third segment 202c relative to the second segment 202b such that the distal surface 204 of the third segment is rotated in a posterior direction (i.e., toward the surface of the lungs "L"). The third segment 202c is rotated to a position where the third segment 202c is incident to the surface of the lungs "L" (i.e., between 0 and 90 degrees relative to the second segment 202b) such that the distal surface 204 is facing the surface of the lungs "L".

Once facing the surface of the lung "L" (i.e., incident the lung surface), the laser 210 emits IR light, which is reflected off the surface of the lungs "L" and detected by the second camera 212. The endoscope is advanced over the surface of the lungs "L" in a caudal, cephalad, or lateral direction, or combinations thereof. The data obtained by the second camera 212 is processed by the computer (not shown) to generate a three dimensional (3D) map of the surface of the lungs "L" using any suitable means, such as stitching or the like. Thus, the clinician advances the endoscope 200 over the entire surface of the lungs "L" in order to obtain a complete a map as possible.

The light source 208 and the optical camera 206 are simultaneously operated with the laser 210 and second camera 212 to permit the clinician to correlate the images received from the optical camera 206 with the previously acquired MRI (or other modality identified above) images. The correlation between the images obtained by the optical camera 206 and the previously acquired MRI images permits the clinician, and the computer, to more accurately map the surface of the lungs "L". As can be appreciated, the accuracy of the correlation may be further improved using tracking software to track the distal tip of the endoscope 200, such as the tracking software described above.

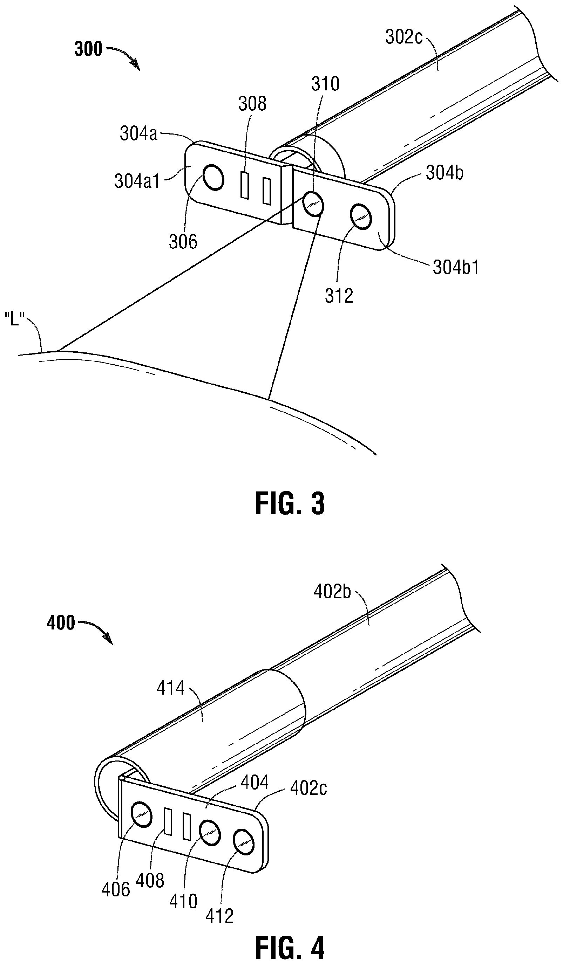

Referring now to FIG. 3, another embodiment of an endoscope having surface scanning capabilities provided in accordance with the present disclosure is illustrated and generally identified by reference numeral 300. Endoscope 300 is substantially similar to that of previously described endoscope 200, and thus, only the differences therebetween will be described for purposed of brevity. The distal end of the third segment 302c of the elongate body 302 includes a pair of articulating jaws 304a and 304b pivotable between a first, approximated position and a second, open position. When placed in the first, approximated position, the outer diameter of the jaws 304a, 304b is substantially similar to that of the third segment 302c, thereby maintaining a small profile to more easily pass within the thoracic cavity and to minimize the size of the incision required. The pair of articulating jaws 304a, 304b are in mechanical communication with an actuation device (not shown) operatable by a clinician, such that the clinician may selectively manipulate the pair of jaws 304a, 304b from the first, approximated portion, to the second, open position.

Each jaw 304a and 304b includes an inner surface 304a1 and 304b1, respectively. The inner surface 304a1 of jaw 304a includes an optical camera 306 and a light source 308 disposed thereon and the inner surface 304b1 of jaw 304b includes a laser 310, and a second camera 312. Each of the optical camera 306, light source 308, laser 310, and IR camera 312 are identical to that of the optical camera 206, light source 208, laser 210, and second camera 212 of the endoscope 200, and therefore will not be described in detail herein. As can be appreciated, the placement of the optical camera 306, light source 308, laser 310, and second camera 312 may be reversed. The optical camera 306, light source 308, laser 310, and second camera 312 are protected by a transparent protective cover (not shown) disposed on each inner surface 304a1 and 304b1, similar to that described above with respect to endoscope 200.

In embodiments, the elongate body 202 may include a fluid tube disposed therein and terminating at the pair of jaws 304a, 304b. The fluid tube is in fluid communication with a fluid reservoir and suitable pump such that a cleaning solution or other suitable fluid may be ejected on to the inner surfaces 304a1, 304b1 of the pair of jaws 304a, 304b to flush and clean soil or blood from the inner surfaces 304a1, 304b1. Similarly Laser 210 may be on a hinged surface or jaw and adjacent a fluid tube to eject cleaning solution onto the laser emitting face.

In operation, endoscope 300 is used in a similar manner to endoscope 200 except that once the third segment 302c has been advanced within the thoracic cavity, the clinician manipulates the pair of jaws 304a, 304b such that the pair of jaws 304a, 304b are caused to rotate from the first, approximated position to the second, open position. The procedure described above with respect to endoscope 200 may then be followed.

With reference to FIG. 4, yet another embodiment of an endoscope having surface scanning capabilities provided in accordance with the present disclosure is illustrated and generally identified by reference numeral 400. Endoscope 400 is substantially similar to that of previously described endoscope 200, and thus, only the differences therebetween will be described for purposed of brevity. The second segment 402b of the elongate body includes a cover 414 slidably secured thereto from a first, extended position where the third segment 402c is disposed therein and a second, retracted position where the third segment 402c is exposed (i.e., no longer shielded by the cover 414). The cover 414 is in mechanical communication with a suitable actuator such that a clinician may selectively manipulate the cover from the first, extended position, to the second, retracted position. The third segment 402c may be independently actuatable by a clinician with respect to the cover 414, or the third segment 402c may be in mechanical communication with the cover 414. In this manner, the third segment 402c may be coupled to the cover 414 by any suitable means, such as wires, rods, etc., such that when the cover is placed in the first, extended position, the cover 414 causes the third segment 402c to be placed in a first position where the third segment 402c is axially aligned within the second segment 402b, and when the cover 414 is placed in the second, retracted position, the cover 414 causes the third segment 402c to rotate to a second position where the third segment 402c is incident to the second segment 402b.

In embodiments, a biasing element (not shown) may be interposed between the third segment 402c and the second segment 402b, biasing the third segment 402c to a second, articulated position. In this manner, when the cover 414 is placed in the first, extended position, the third segment 402c is fully received within the cover 414 and inhibited from articulated (i.e., axially aligned with the second segment). When the cover 414 is placed in the second, retracted position, the biasing element biases the third segment 402c to the second, articulated position (i.e., rotated to an angle incident with the second segment 402b). As can be appreciated, the biasing element may be placed in tension or compression, and may be any biasing element known in the art, such as coil spring, leaf spring, or the like.

The third segment includes an outer surface 404 on which an optical camera 406, light source 408, laser 410, and second camera 412 are disposed. Each of the optical camera 406, light source 408, laser 410, and second camera 412 are identical to that of the optical camera 206, light source 208, laser 210, and second camera 212 of the endoscope 200, and therefore will not be described in detail herein. As can be appreciated, the optical camera 406, light source 408, laser 410, and second camera 412 may be disposed in any position, order, or orientation. It is envisioned that the endoscope 400 may include a fluid tube (not shown) as described with respect to endoscope 300, such that the fluid tube may eject fluid onto the

In operation, endoscope 400 is used in a similar manner to endoscope 200 except that once the third segment 302c has been advanced within the thoracic cavity, the clinician manipulates the cover 414 from the first, extended position to the second, retracted position to expose the outer surface 404 of the third segment 402c. The retraction of the cover 414 causes the third segment 402c to rotate to the second, articulated position, at which point the procedure described above with respect to endoscope 200 may be followed.

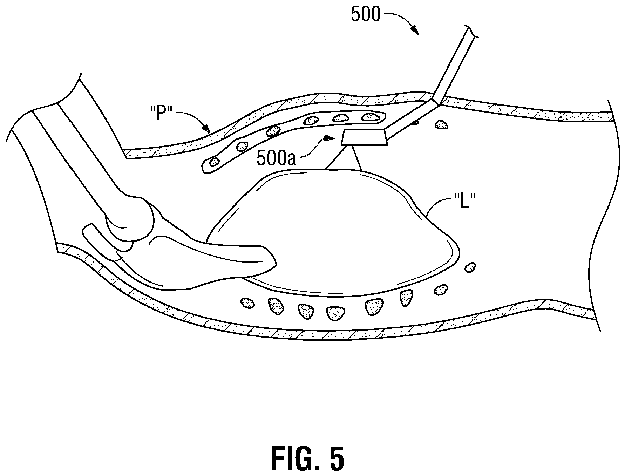

Turning now to FIGS. 5-7, another embodiment of an endoscope having surface scanning capabilities provided in accordance with the present disclosure is illustrated and generally identified by reference numeral 500. The endoscope 500 is substantially similar to that of previously described endoscope 200, and thus, only the differences therebetween will be described for purposes of brevity. A distal portion 500a of the endoscope 500 includes a projection assembly 502 and a camera 504 disposed therein. As can be appreciated, the distal portion 500a of the endoscope 500 may be similar to the third segment 202c described in detail above with respect to endoscope 200.

The projection assembly 502 is a digital light processing (DLP) projection system and includes a DLP chip 502a, a light source 502b, one or more mirrors 502c, a first lens 502d, and a second lens 502e, although it is contemplated that the projection assembly 502 may include any suitable components capable of projecting DLP images. In embodiments, the DLP projection system may be a single-chip or three-chip system, and in one non-limiting embodiment, may be selected from the pico category (ultra-small). The light source 502b may be any suitable light source capable of projecting DLP images, such as LED, laser, halogen, or the like.

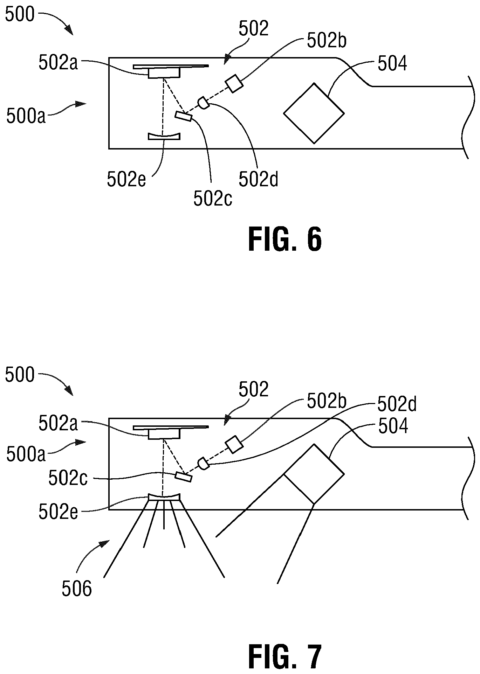

As best illustrated in FIG. 7, the light source 502b emits light towards the first lens 502d. The first lens 502d aligned with the light source 502b and is configured to collimate light emitted from the light source 502b (i.e., align diverging/converging light parallel to each other and normal to the lens). The first lens 502d may be a collimating lens (i.e., plano-convex) having a planar surface and an opposite, convex surface, although any suitable arrangement is contemplated. The mirror 502c is disposed within the projection assembly 502 and is oriented at an acute angle with respect to the light source 502b and the first lens 502d. As can be appreciated, the angle at which the mirror 502c is oriented relative to the light source 502b and the first lens 502d may be adjusted depending upon the location of the DLP chip 502a, the light source 502b, and the first lens 502d within the projection assembly 502.

The mirror 502c reflects the collimated light exiting the first lens 502d towards the DLP chip 502a. The DLP chip 502a processes the light reflected by the mirror 502c and reflects the light towards and through the second lens 502e. Although generally illustrated as being a plano-concave lens, it is contemplated that the second lens 502e may be any suitable lens capable of focusing and/or redirecting the light in any suitable pattern. As can be appreciated, the profile of the second lens 502e may be selected based upon the needs of the procedure being performed. As best illustrated in FIG. 7, the second lens 502e projects the image pattern generated by the DLP chip 502a at a generally perpendicular angle relative to a longitudinal axis defined by the elongate body 202, although it is contemplated that any suitable orientation may be utilized.

The DLP chip 502a is configured to project a customizable structured light pattern 506 onto the anatomy of the patient (FIG. 5). It is contemplated that the structured light pattern 506 may be tailored to the specific anatomy of the patient that is being scanned, such as projecting over a large or small field of view, a narrow field or view, or the like. As can be appreciated, the DLP chip 502a is capable of generating myriad images having myriad shapes, intensity, coverage area, or the like, that may be projected onto the patient's anatomy. In one non-limiting embodiment, the DLP chip 502a may project a structured light pattern 506 matching the overall shape of the portion of the patient's anatomy that is being scanned (e.g., oval, circular, irregular, or the like).

The camera 504 is disposed within the distal end portion 500a of the endoscope 500 at a known angle and distance from the second lens 502e such that the structured light pattern 506 projected by the DLP chip 502a may be accurately identified. The known distance and angle of the camera 504 relative to the second lens 502e is entered into and stored within a memory (not shown) coupled to the computer (not shown) along with suitable mathematical relationships (such as trigonometric functions or other suitable logarithms). The known distance and angle of the camera 504 relative to the second lens 502e are entered into the mathematical relationships along with the known structured light pattern 506 being projected by the DLP chip 502a. Using these mathematical relationships, the precise depth and location of each portion of the image can be calculated and the topography of the patient's anatomy may be determined at a plurality of locations at once, covering a large field of view or area, rather than at a singular point or more narrow field of view.

It is contemplated that the structured light pattern 506 projected by the DLP chip 502a may be altered or customized to correct for optical aberrations or distortions resulting from the second lens 502e or any other optical device within the path of light emitted from the light source 502b (such as the first lens 502d, the mirror 502c, or the like). In this manner, it is contemplated that the DLP chip 502a may project a pre-distorted image to compensate for measured distortion through the optical devices (e.g., the first lens 502d, the mirror 502c, and the second lens 502e) within the projection assembly 502.

As can be appreciated, the projection assembly 502 and the camera 504 may be electrically coupled to the computer, processor, and memory (not shown) associated with the endoscope 500. In this manner, it is contemplated that a clinician may enter in commands or control the structured light pattern 506 projected by the DLP chip 502a using any suitable user input device (i.e., touchscreen, mouse, keyboard, or the like).

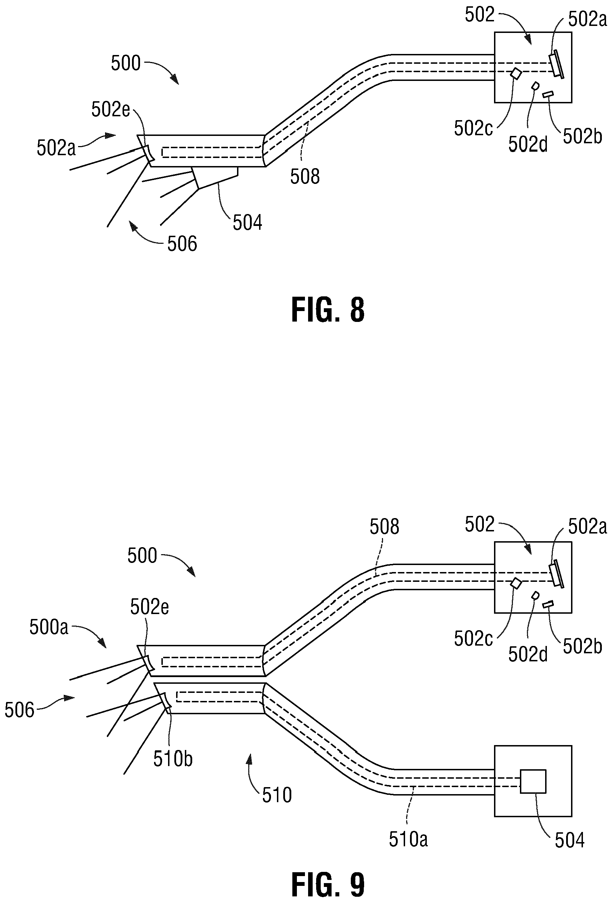

As illustrated in FIG. 8, in embodiments, the projection assembly 502 may be disposed remote from the distal end portion 500a of the endoscope 500. In this manner, the projection assembly 502 is disposed external to the patient and the structured light pattern 506 projected by the projection assembly 502 is transmitted through a light guide 508, such as a fiber optic cable, coupled to the DLP chip 502a or any suitable lens (not shown) associated with the output of the DLP chip 502a. It is contemplated that the light guide may be formed from substantially hollow members with mirrors disposed at flex points defined along the length of the substantially hollow members, as is commonly known. Alternatively, it is contemplated that the light guide may be formed as a solid flexible fiber or a hollow flexible fiber, depending upon the wavelength of the structured light required. Although generally referred to as being a light guide cable, it is contemplated that any suitable means of transmitting light or optical information may be utilized depending upon the needs of the procedure being performed.

The light guide 508 terminates at the second lens 502e which is oriented at an angle relative to the longitudinal axis of the elongate member 202. Although generally shown as defining a 45 degree angle relative to the longitudinal axis, it is contemplated that the second lens 502e may be oriented at any suitable angle relative to the longitudinal axis. The camera 504 is disposed on the distal portion of the endoscope 500 and is oriented parallel to the longitudinal axis. In this manner, the camera 504 is in a position to intercept and capture the reflected structured light pattern 506. It is contemplated that the camera may be disposed within the elongate body 202 or may be disposed on an exterior portion of the elongate body 202 using any suitable means, such as fasteners, adhesives, overmolding, or the like.

As can be appreciated, by locating the projection assembly 502 external to the patient, the endoscope 500 may be comparatively reduced in size. Additionally, the size of the DLP chip 502a is not constrained by the size of the endoscope 500. Therefore, any suitable DLP projector may be utilized along with any suitable light source 502b without concern for minimizing. As can be appreciated, cleaning the endoscope 500 is simplified as well as only the endoscope 500 requires cleaning rather than the entire DLP projection system in addition to the endoscope 500.

With reference to FIG. 9, it is contemplated that the camera 504 may be coupled to a second endoscope 510 that is separate and distinct from the endoscope 500, rather than coupled to the endoscope 500 as described hereinabove. It is contemplated that the camera 504 may be disposed within a distal end portion 510a of the second endoscope 510, or in one non-limiting embodiment, may be disposed external to the patient in a similar manner as described above with respect to the projection assembly 502.

The second endoscope 510 is similar to endoscope 500 and includes a light guide 510a or other similar means of transmitting light or optics from the projection assembly 502. The light guide 510a terminates at a lens 510b capable of focusing the camera 504. It is contemplated that the lens 510b may be configured to focus the camera 504, or in embodiments, the lens 510b may be fixed. Although generally illustrated as being oriented at an angle with respect to a longitudinal axis "X" defined by the endoscope 510, it is contemplated that the lens 510b may be disposed at any suitable angle relative to the longitudinal axis "X" depending upon the needs of the procedure being performed. Although generally illustrated as being fixedly secured to the endoscope 500, it is contemplated that the second endoscope may be advanced within the patient at the same or different location relative to the endoscope 500. In this manner, the projection assembly 502 and the camera 502 may have the same or different fields of view. Additionally, by placing the camera 502 external to the patient, the second endoscope 510 may be comparatively reduced in size. Further, the type and size of the camera 504 is not constrained by the size of the second endoscope 510. In addition, only the second endoscope 510 needs to be cleaned after a procedure rather than the camera electronics in addition to the second endoscope 510.

As can be appreciated, the location at which the lens 510b is located relative to the second lens 502e of the endoscope 500 must be known in order to accurately calculate the topography of the patient's anatomy. If the second endoscope 510 is fixedly secured to the endoscope 500, the location of the lens 510b relative to the second lens 502e is always known. However, if the second endoscope 510 is advanced within the patient separate from the endoscope 500, the location of the lens 510b relative to the second lens 502e must be determined. It is contemplated that any suitable means for determining the location of each of the lens 510b and the second lens 502e may be utilized, such as electromagnetic sensors, imaging modalities such as MRI, Flouroscopy, etc., RFID, or the like.

In operation, initially, the patient "P" (FIG. 5) is imaged using any suitable imaging device (not shown), such as MRI, ultrasound, CT scan, Positron Emission Tomography (PET), or the like, and the images are stored within the memory (not shown) coupled to the computer (not shown). After the patient "P" is imaged, the clinician penetrates the chest of the patient "P" using a trocar (not shown) or other suitable device. The endoscope 500 is advanced within the trocar, and thereafter, within the thoracic cavity of the patient "P" (FIG. 5). As the endoscope 500 is further advanced within the thoracic cavity, the clinician observes the images obtained by the camera 504 on a display (not shown), and once the distal end portion 500a of the endoscope 500 is adjacent the lungs "L," the clinician manipulates the endoscope 500 to orient the distal end portion 500a of the endoscope 500 to an orientation that is parallel relative to the surface of the lungs "L" (FIG. 5). Once the distal end portion 500a of the endoscope 500 is in a parallel orientation, the clinician activates the projector assembly 502 and projects the structured light pattern 506 onto the surface of the lungs "L."

The structured light pattern 506 is reflected off of the surface of the lungs "L" and is detected by the camera 504. The distal end portion 500a of the endoscope 500 is advances over the surface of the lungs "L" in a caudal direction, a cephalad direction, a lateral direction, or combinations thereof. The data obtained by the camera 504, such as the known distance and angle of the camera 504 relative to the second lens 502e and the known structured light pattern 506 projected by the projection assembly 502, is processed by the computer (not shown) to generate a three-dimensional (3D) map of the surface of the lungs "L" using any suitable means, such as stitching or the like. Thus, the clinician advances the endoscope 500 over the entire surface of the lungs "L" in order to obtain a complete a map of the surface of the lungs "L" as possible.

Surgical instruments such as the endoscopes described herein may also be configured to work with robotic surgical systems and what is commonly referred to as "Telesurgery." Such systems employ various robotic elements to assist the surgeon and allow remote operation (or partial remote operation) of surgical instrumentation. Various robotic arms, gears, cams, pulleys, electric and mechanical motors, etc. may be employed for this purpose and may be designed with a robotic surgical system to assist the surgeon during the course of an operation or treatment. Such robotic systems may include remotely steerable systems, automatically flexible surgical systems, remotely flexible surgical systems, remotely articulating surgical systems, wireless surgical systems, modular or selectively configurable remotely operated surgical systems, etc.

The robotic surgical systems may be employed with one or more consoles that are next to the operating theater or located in a remote location. In this instance, one team of surgeons or nurses may prep the patient for surgery and configure the robotic surgical system with one or more of the instruments disclosed herein while another surgeon (or group of surgeons) remotely control the instruments via the robotic surgical system. As can be appreciated, a highly skilled surgeon may perform multiple operations in multiple locations without leaving his/her remote console which can be both economically advantageous and a benefit to the patient or a series of patients.

The robotic arms of the surgical system are typically coupled to a pair of master handles by a controller. The handles can be moved by the surgeon to produce a corresponding movement of the working ends of any type of surgical instrument (e.g., end effectors, graspers, knifes, scissors, endoscopes, etc.) which may complement the use of one or more of the embodiments described herein. The movement of the master handles may be scaled so that the working ends have a corresponding movement that is different, smaller or larger, than the movement performed by the operating hands of the surgeon. The scale factor or gearing ratio may be adjustable so that the operator can control the resolution of the working ends of the surgical instrument(s).

It is contemplated that the endoscopes described herein may be positioned by the robotic system and the precise position of the endoscope transmitted to the computer to construct the 3D image of the scanned organ or operative field. The robotic system has the ability to autonomously scan the surgical field and construct a complete 3D model of the field to aid the surgeon in directing the robotic arms or to provide necessary 3D information for the robotic system to further conduct surgical steps autonomously. In embodiments, where the endoscope includes a camera and a structured light source that are independent of one another, the robotic system may direct the camera and a structured light source separately. The robotic system provides the relative coordinates between respective endoscopes needed to triangulate the points in the structured light and camera views to construct a 3D surface of the operative field. In this manner, the robotic system has a specific advantage of being able to autonomously position the structure light source onto the field of view of the camera or camera endoscope.

The master handles may include various sensors to provide feedback to the surgeon relating to various tissue parameters or conditions, e.g., tissue resistance due to manipulation, cutting or otherwise treating, pressure by the instrument onto the tissue, tissue temperature, tissue impedance, etc. As can be appreciated, such sensors provide the surgeon with enhanced tactile feedback simulating actual operating conditions. The master handles may also include a variety of different actuators for delicate tissue manipulation or treatment further enhancing the surgeon's ability to mimic actual operating conditions.

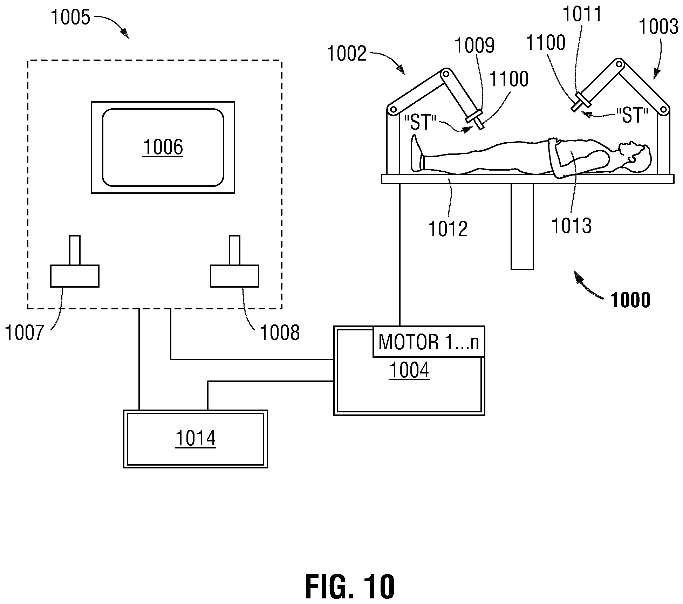

Referring to FIG. 10, a medical work station is shown generally as work station 1000 and generally may include a plurality of robot arms 1002, 1003; a control device 1004; and an operating console 1005 coupled with control device 1004. Operating console 1005 may include a display device 1006, which may be set up in particular to display three-dimensional images; and manual input devices 1007, 1008, by means of which a person (not shown), for example a surgeon, may be able to telemanipulate robot arms 1002, 1003 in a first operating mode.

Each of the robot arms 1002, 1003 may include a plurality of members, which are connected through joints, and an attaching device 1009, 1011, to which may be attached, for example, a surgical tool "ST" supporting an end effector 1100, in accordance with any one of several embodiments disclosed herein, as will be described in greater detail below.

Robot arms 1002, 1003 may be driven by electric drives (not shown) that are connected to control device 1004. Control device 1004 (e.g., a computer) may be set up to activate the drives, in particular by means of a computer program, in such a way that robot arms 1002, 1003, their attaching devices 1009, 1011 and thus the surgical tool (including end effector 1100) execute a desired movement according to a movement defined by means of manual input devices 1007, 1008. Control device 1004 may also be set up in such a way that it regulates the movement of robot arms 1002, 1003 and/or of the drives.

Medical work station 1000 may be configured for use on a patient 1013 lying on a patient table 1012 to be treated in a minimally invasive manner by means of end effector 1100. Medical work station 1000 may also include more than two robot arms 1002, 1003, the additional robot arms likewise being connected to control device 1004 and being telemanipulatable by means of operating console 1005. A medical instrument or surgical tool (including an end effector 1100) may also be attached to the additional robot arm. Medical work station 1000 may include a database 1014, in particular coupled to with control device 1004, in which are stored, for example, pre-operative data from patient/living being 1013 and/or anatomical atlases.

Reference is made herein to U.S. Patent No. 8,828,023, to Thomas Neff et al., entitled "Medical Workstation," the entire content of which is incorporated herein by reference, for a more detailed discussion of the construction and operation of an exemplary robotic surgical system.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments.

As used hereinabove, the term "clinician" refers to a doctor, a nurse, or any other care provider and may include support personnel. Throughout this description, the term "proximal" refers to the portion of the device or component thereof that is closer to the clinician and the term "distal" refers to the portion of the device or component thereof that is farther from the clinician. Further, the term "caudal" refers to a direction towards the patient's feet and the term "cephalad" refers to a direction towards the patient's head. Additionally, in the drawings and in the description above, terms such as front, rear, upper, lower, top, bottom, and similar directional terms are used simply for convenience of description and are not intended to limit the disclosure. In the description hereinabove, well-known functions or constructions are not described in detail to avoid obscuring the present disclosure in unnecessary detail.

**Aspects of the invention are set out in the following numbered paragraphs which are part of the description**
1. A surgical apparatus comprising:
   an endoscope including an elongate body, the elongate body including a plurality of segments manipulatable relative to one another;
   a camera;
   a light source; and
   a structured light pattern source,
   wherein the camera, the light source, and the structured light pattern source cooperate to determine the topography of a surface within a patient.
2. The surgical apparatus according to paragraph 1, wherein the structured light pattern source is a structured light scanning laser.
3. The surgical apparatus according to paragraph 2, wherein the structured light scanning laser is an LED laser having collimated light, the collimated light being in the infrared or visible spectrum.
4. The surgical apparatus according to paragraph 2, further including a second camera configured to intercept reflected light from the structured light scanning laser.
5. The surgical apparatus according to paragraph 4, wherein a distal-most segment of the endoscope terminates at a distal face, wherein the camera, the light source, the structured light scanning laser, and the second camera are disposed within the distal face of the elongate body.
6. The surgical apparatus according to paragraph 4, wherein a distal-most segment of the endoscope terminates at a distal face, wherein the camera, the light source, and the second camera are disposed within the distal face and the structured light scanning laser is disposed on an outer surface of the distal-most segment.
7. The surgical apparatus according to paragraph 6, wherein the structured light scanning laser and the second camera are oriented at an angle relative to a centerline defined by the distal most segment.
8. The surgical apparatus according to paragraph 1, wherein the structured light pattern source is a digital light processing system.
9. The surgical apparatus according to paragraph 8, wherein the digital light processing system includes a light source, at least one mirror, a first lens, a digital light processing chip, and a second lens.
10. The surgical apparatus according to paragraph 8, wherein the digital light processing system and the camera are disposed within the distal-most segment of the endoscope.
11. The surgical apparatus according to paragraph 8, wherein the digital light processing system is disposed external to the distal-most portion of the endoscope and the camera is disposed within the distal-most segment of the endoscope.
12. The surgical apparatus according to paragraph 8, wherein the digital light processing system and the camera are disposed external to the distal-most segment of the endoscope.
13. The surgical apparatus according to paragraph 8, further including a second endoscope, wherein the digital light processing system is coupled to the endoscope and the camera is coupled to the second endoscope.
14. A method of performing surgery, comprising:
   storing instructions on a memory associated with a computer;
   advancing an endoscope within a body cavity of a patient;
   manipulating a distal segment of a plurality of segments defined by the endoscope towards a target tissue;
   projecting a structured light pattern from a structured light pattern source coupled to the distal segment;
   detecting portions of the structured light pattern that is reflected off of the target tissue using a camera coupled to the distal segment; and
   executing the instructions to generate a three dimensional map of the target tissue using data obtained by the camera.
15. The method according to paragraph 14, wherein projecting a structured light pattern from a structured light pattern source includes projecting a structured light pattern from a structured light scanning laser.
16. The method according to paragraph 15, wherein detecting portions of the structured light pattern includes detecting portions of the structured light pattern using a second camera coupled to the distal segment.
17. The method according to paragraph 14, wherein projecting a structured light pattern from a structured light pattern source includes projecting a structured light pattern from a digital light processing system.
18. The method according to paragraph 14, further including correlating images obtained by an optical camera coupled to the distal segment with images of a patient stored on the memory.
19. The method according to paragraph 18, further including tracking a location of the endoscope within the body cavity of the patient.
20. The method according to paragraph 18, wherein executing the instructions to generate a three dimensional map of the target tissue includes utilizing the correlated images and the tracked location of the endoscope to generate an accurate three dimensional map of the target tissue.

## Claims

1. A surgical apparatus comprising:
an endoscope including an elongate body, the elongate body including a plurality of segments manipulatable relative to one another;
a camera;
a light source; and
a structured light pattern source,
wherein the camera, the light source, and the structured light pattern source cooperate to determine the topography of a surface within a patient.

2. The surgical apparatus according to claim 1, wherein the structured light pattern source is a structured light scanning laser, preferably wherein the structured light scanning laser is an LED laser having collimated light, the collimated light being in the infrared or visible spectrum.

3. The surgical apparatus according to claim 1 or 2, further including a second camera configured to intercept reflected light from the structured light scanning laser.

4. The surgical apparatus according to claim 3, wherein a distal-most segment of the endoscope terminates at a distal face, wherein the camera, the light source, the structured light scanning laser, and the second camera are disposed within the distal face of the elongate body; or
wherein a distal-most segment of the endoscope terminates at a distal face, wherein the camera, the light source, and the second camera are disposed within the distal face and the structured light scanning laser is disposed on an outer surface of the distal-most segment.

5. The surgical apparatus according to claim 4, wherein the structured light scanning laser and the second camera are oriented at an angle relative to a centerline defined by the distal most segment.

6. The surgical apparatus according to claim 1, wherein the structured light pattern source is a digital light processing system.

7. The surgical apparatus according to claim 6, wherein the digital light processing system includes a light source, at least one mirror, a first lens, a digital light processing chip, and a second lens.

8. The surgical apparatus according to claim 6, wherein the digital light processing system and the camera are disposed within the distal-most segment of the endoscope; or
wherein the digital light processing system is disposed external to the distal-most portion of the endoscope and the camera is disposed within the distal-most segment of the endoscope; or
wherein the digital light processing system and the camera are disposed external to the distal-most segment of the endoscope.

9. The surgical apparatus according to claim 6, further including a second endoscope, wherein the digital light processing system is coupled to the endoscope and the camera is coupled to the second endoscope.

10. A system for generating a map of tissue, comprising:
a memory associated with a computer, the memory storing instructions;
an endoscope for advancing within a body cavity of a patient;
the endoscope having a plurality of segments of which a distal segment is manipulatable towards a target tissue;
a structured light pattern source coupled to the distal segment for projecting a structured light pattern; and
a camera coupled to the distal segment for detecting portions of the structured light pattern that is reflected off of the target tissue using; and
the computer being configured to execute the instructions to generate a three dimensional map of the target tissue using data obtained by the camera.

11. The system according to claim 10, wherein the structured light pattern source comprises a structured light scanning laser; or wherein the structured light pattern source comprises a digital light processing system.

12. The system according to claim 11, comprising a second camera coupled to the distal segment for detecting portions of the structured light pattern.

13. The system according to claim 10, further including means for correlating images obtained by an optical camera coupled to the distal segment with images of a patient stored on the memory.

14. The system according to claim 13, further including means for tracking a location of the endoscope within the body cavity of the patient.

15. The system according to claim 13, wherein the computer is configured to execute the instructions to generate a three dimensional map of the target tissue utilizing the correlated images and the tracked location of the endoscope to generate an accurate three dimensional map of the target tissue.
